(19) Europäisches Patentamt
European Patent Office
Office européen des brevets

(11) EP 4 076 353 B1

(12) # EUROPÄISCHE PATENTSCHRIFT

(45) Veröffentlichungstag und Bekanntmachung des Hinweises auf die Patenterteilung:
**11.03.2026 Patentblatt 2026/11**

(21) Anmeldenummer: **20808342.8**

(22) Anmeldetag: **17.11.2020**

(51) Internationale Patentklassifikation (IPC):
***A61K 8/19*** (2006.01) ***A61Q 5/06*** (2006.01)
***A61K 8/44*** (2006.01) ***A61K 8/34*** (2006.01)
***A61Q 5/12*** (2006.01) ***A61K 8/92*** (2006.01)

(52) Gemeinsame Patentklassifikation (CPC):
**A61K 8/442; A61K 8/19; A61K 8/342; A61K 8/922; A61Q 5/065; A61Q 5/12**

(86) Internationale Anmeldenummer:
**PCT/EP2020/082331**

(87) Internationale Veröffentlichungsnummer:
**WO 2021/121823 (24.06.2021 Gazette 2021/25)**

(54) **TÖNUNGSCONDITIONER MIT VERBESSERTER FÄRBELEISTUNG**

TINTING CONDITIONER HAVING IMPROVED DYEING PERFORMANCE

APRÈS-SHAMPOING COLORANT AYANT UNE PERFORMANCE AMÉLIORÉE DE COLORATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.12.2019 DE 102019219650**

(43) Veröffentlichungstag der Anmeldung:
**26.10.2022 Patentblatt 2022/43**

(73) Patentinhaber: **Henkel AG & Co. KGaA**
**40589 Düsseldorf (DE)**

(72) Erfinder:
- **GROSJACQUES, Camille**
  **20255 Hamburg (DE)**
- **HEIDENREICH, Katharina**
  **22041 Hamburg (DE)**

(56) Entgegenhaltungen:
**WO-A1-02/02062**
**WO-A2-02/100363**
**DE-A1- 102011 089 223**
**DE-A1- 102017 223 420**

- **"Henne Color Creme Colorante", 28 June 2009 (2009-06-28), XP055000954, Retrieved from the Internet <URL:http://www.codecheck.info/kosmetischemittel/haarpflege/haarfaerbemi...> [retrieved on 20110620]**
- **"Hair dyeing composition ED - Darl Kuhn", IP.COM, IP.COM INC., WEST HENRIETTA, NY, US, 15 July 2009 (2009-07-15), XP013132810, ISSN: 1533-0001**
- **"CONDITIONING AGENTS FOR OPTIMISED HAIR COLOUR", INTERNET CITATION, 2006, XP002462066, Retrieved from the Internet <URL:http://personalcaremagazine.com/Print.aspx?Story=720> [retrieved on 20071212]**

EP 4 076 353 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung liegt auf dem Gebiet der Kosmetik und betrifft Tönungsconditioner zum gleichzeitigen semipermanenten Färben und Pflegen von Haaren, die mindestens einen direktziehenden Farbstoff sowie eine Kombination aus mindestens einem Fettalkohol, mindestens einem Alkylamidoalkylbetain und mindestens einem wasserlösliches Magnesiumsalz in einer solchen Menge enthalten, dass der Gehalt an $Mg^{2+}$-Ionen 0,005 bis 1,000 Gew.-%, bezogen auf das Gewicht des Tönungsconditioners, beträgt. Diese Tönungsmittel eignen sich zur Pflege und zum Konditionieren der Haare, insbesondere zur Glättung der aufgerauten Haaroberfläche und gesplisster Haarspitzen und gleichzeitig besonders gut zur

- Verbesserung der Waschechtheit der Färbung auf den Haaren und/oder
- Erhöhung der Chromatizität der Färbung auf den Haaren und/oder
- Erhöhung des Farbaufzugs der Farbstoffe auf die Haare und/oder
- Erhöhung der Farbintensität der Färbung auf den Haaren.

Die spezielle Kombination an Inhaltsstoffen in der erfindungsgemäßen Fettalkoholemulsion als kosmetischem Träger, insbesondere die Kombination aus amphoterem Tensid und Magnesiumsalz gemäß Anspruch 1, erzielte sehr gute Färberesultate in Bezug auf die Waschechtheit sowie die Färbungseigenschaften, wie Chromatizität und Farbintensität. Im Stand der Technik waren Mittel zur semipermanenten Haarfärbung, die ein Alkylamidoalkylbetain, insbesondere ein Alkylamidopropylbetain, enthalten, bereits bekannt. Der Gehalt an wasserlöslichem Magnesiumsalz, wie insbesondere Magnesiumchlorid oder Trimagnesiumdicitrat, leistet einen überraschenden Beitrag zur Verbesserung der Echtheitseigenschaften der Färbung wie auch zu den Haar konditionierenden Eigenschaften.

Tönungsconditioner sind zurzeit bei den Verbrauchern eine ausgesprochen beliebte Produktgruppe, da sie den Wunsch nach Auffrischung einer bereits bestehenden Färbung, die meist eine permanente Oxidationsfärbung ist, befriedigen, ohne die Haare zu schädigen und sie im Gegenteil noch mit einer besonderen Pflege zu versehen. Außerdem vereint diese Produktgruppe zwei Funktionen in einem Produkt, nämlich das Färben und das Konditionieren des Haars. Haarconditioner wie auch Tönungsmittel, das heißt, direktziehende Färbemittel, benötigen eine gewisse Einwirkzeit. Aus Gründen der Zeitersparnis ist es für den Verbraucher attraktiv, beide Funktionen in einem einzigen Produkt vereint zu haben.

Darüber hinaus gestattet es die semipermanente Färbung mit direktziehenden Farbstoffen aufgrund der im Vergleich zu oxidativen Färbungen schnelleren Auswaschbarkeit, häufiger die Haarfarbe zu variieren.

Auf der Internetseite http://www.codecheck.info/kosmetischemittel/haarpflege / haarfaerbemi...., wurde am 20.06.2011 eine Tönungscreme namens Henné Color Crème Colorante publiziert, die Wasser, Cetearylalkohol, Propylenglykol, Ceteareth-33, Cocamidopropylbetain, Cetrimoniumchlorid, Quaternium-52, Lawsonia Inermis (Henna)-Extrakt, PEG-15-Cocopolyamin, Lavandula-Hybrida-Öl, Natriumchlorid, Magnesiumnitrat, Methylchloroisothiazolin, Hydroxyethyl-2-nitro-p-toluidin, HC Red Nr. 3 und Linalool enthält. Die PriorArtDatabase IP.com offenbart unter IPCOM000185206D Färbemittel, die mindestens zwei kationische direktziehende Farbstoffe enthalten, darunter den kationischen Direktzieher Basic Blue 124. Die Offenlegungsschrift WO 2002/02062A1 beschäftigt sich mit der Verbesserung des Farbaufzugs von anionischen direktziehenden Farbstoffen auf das Haar durch den Zusatz von Salzen, darunter auch Magnesiumsalzen. Die Offenlegungsschrift DE102011089223A1 beschäftigt sich mit Färbemitteln, die zwingend den kationischen direktziehenden Farbstoff Cationic Blue 347 enthalten. Die Offenlegungsschrift DE102017223420A1 beschäftigt sich mit der Verbesserung von temporären Färbemitteln auf der Basis von Direktziehern, wobei Alkylglycoside einen wesentlichen Bestandteil dieser Mittel darstellen. Die Offenlegungsschrift WO2002100363A2 und die Internetpublikation http://personalcaremagazine.com/Print.aspx?Story=720 beschäftigen sich ebenfalls nur mit Färbemitteln, die zwingend einen kationischen direktziehenden Farbstoff enthalten.

Als Rahmenbedingung für die Lösung der gestellten Aufgaben galt, als Basis für den erfindungsgemäßen Tönungsconditioner eine Fettalkohol-haltige Emulsion zu verwenden, um sich die bewährten Haarkonditionierenden Eigenschaften dieses Trägermaterials zunutze zu machen. Bei der Produktentwicklung galt es, eine Balance zu finden zwischen akzeptablen Gebrauchseigenschaften, insbesondere eine gute Auswaschbarkeit des Produktes nach Ablauf der Einwirkzeit, ein gutes Farbaufzugsvermögen auf die Haarfasem, eine lang anhaltende Färbung, eine hohe Waschechtheit und zusätzlich dazu gute Haarkonditionierende Eigenschaften, denn viele Verbraucher verwenden einen solchen Tönungsconditioner auf ihren Haaren, die bereits durch ein oxidative Haarfärbung oder Blondierung vorgeschädigt sind.

Die Veränderung von Form und Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, so genannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch hervorragende, langanhaltende Färbeergebnisse aus, sind jedoch auch mit einem gewissen Ausmaß an Haarschädigung verbunden.

Möchte der Anwender Haarschädigungen reduzieren oder die Haarfarbe nur temporär verändern, kann er auf Färbemittel zurückgreifen, die auf direktziehenden Farbstoffen basieren. Hierbei diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Auch die Grauabdeckungen, die mit direktziehenden Farbstoffen erhalten werden können, sind in der Regel verbesserungswürdig. Von Vorteil ist jedoch die geringere Haarschädigung der Färbung mit direktziehenden Farbstoffen.

Abhängig vom gewünschten Farbergebnis setzt der Fachmann direktziehende Farbstoffe unterschiedlicher Farbstoffklassen ein. Die aus dem Stand der Technik bekannten direktziehenden Farbstoffe gehören beispielsweise zur Klasse der Nitrofarbstoffe, der Anthrachinonfarbstoffe, der Azofarbstoffe, der Triarylmethanfarbstoffe oder der Methinfarbstoffe. Alle diese Farbstoffklassen sollten für die Anwendung im Kosmetikbereich ein bestimmtes Anforderungsprofil erfüllen. So sollten direktziehende Farbstoffe ein intensives Färbeergebnis liefern und möglichst gute Echtheitseigenschaften besitzen. Durch Umwelteinflüsse sollte das mit direktziehenden Farbstoffen erhaltene Farbergebnis möglichst wenig beeinflusst werden, d.h. die Farbstoffe sollten beispielsweise eine gute Waschechtheit, Lichtechtheit und Reibechtheit besitzen. Auch chemische Einflüsse, welchen die keratinischen Fasern nach dem Färbeprozess ausgesetzt sein können (wie beispielsweise Dauerwellen), sollten das Farbergebnis möglichst wenig verändern.

Direktziehende Farbstoffe können in anionische, kationische und nicht-ionische direktziehende Farbstoffe unterteilt werden. In kosmetischen Zusammensetzungen treten diese in unterschiedlicher Weise in Wechselwirkung mit den übrigen Bestandteilen der Zusammensetzungen. Einen wesentlichen Einfluss haben hierbei enthaltene oberflächenaktive Wirkstoffe wie Tenside bzw. Emulgatoren. Diese sollen in den kosmetischen Zusammensetzungen die keratinischen Fasern von Verunreinigungen befreien und/oder Bestandteile der Zusammensetzung selbst emulgieren. Dabei kann es je nach Auswahl der oberflächenaktiven Substanz jedoch auch zu Wechselwirkungen zwischen diesen und den direktziehenden Farbstoffen kommen, wodurch mitunter das Färbeergebnis im Vergleich zu Mitteln, die keine oberflächenaktiven Substanzen enthalten, deutlich verschlechtert wird.

Tenside bzw. Emulgatoren sind amphiphile (bifunktionelle) Verbindungen, die aus mindestens einem hydrophoben und mindestens einem hydrophilen Molekülteil bestehen. Bei dem hydrophoben Rest handelt es sich üblicherweise um eine Kohlenwasserstoffkette. Der hydrophile Rest kann je nach Art des Tensids eine negative Ladung, eine positive Ladung, eine negative und eine positive Ladung oder keine Ladung aufweisen.

Bei anionischen Tensiden umfasst der hydrophile Molekülteil mindestens eine negativ geladene hydrophile Kopfgruppe. Anionische Tenside enthalten ausschließlich negative Ladungen.

Unter kationischen Tensiden werden Tenside, also grenzflächenaktive Substanzen, mit jeweils einer oder mehreren positiven Ladungen verstanden. Kationische Tenside enthalten ausschließlich positive Ladungen.

Zwitterionische (amphotere) Tenside umfassen in dem hydrophilen Molekülteil mindestens eine negativ geladene und mindestens eine positive geladene Gruppe. Diese sind räumlich voneinander getrennt und liegen nebeneinander vor, wobei das Tensid insgesamt elektrisch neutral ist. Darüber hinaus gibt es nicht-ionische (nicht-ionogene) Tenside, welche sich durch die Abwesenheit elektrischer Ladungen in den Molekülen auszeichnen.

Der vorliegenden Erfindung lag daher die Aufgabe zugrunde, ein pflegendes Haarfärbemittel zu semipermanenten Färbung mit verbesserter Waschechtheit bereitzustellen.

Überraschenderweise hat sich nun herausgestellt, dass die Waschechtheit von semipermanenten Färbungen mit mindestens einem direktziehenden Farbstoff aus einer Fettalkohol-in-Wasser-Emulsion heraus verbessert werden kann, wenn mindestens ein Alkylamidoalkylbetain in einer Gesamtmenge von 0,05 - 3 Gew.-% und mindestens ein wasserlösliches Magnesiumsalz in einer solchen Menge, dass der Gehalt an $Mg^{2+}$-Ionen 0,005 bis 1,000 Gew.-% beträgt, jeweils bezogen auf das Gewicht des Tönungs- oder Färbemittels, enthalten sind.

Ein erster Gegenstand der vorliegenden Erfindung ist ein kosmetisches Mittel zum gleichzeitigen semipermanenten Färben und Pflegen von Haaren gemäß Anspruch 1, enthaltend

(a) mindestens einen direktziehenden Farbstoff, ausgewählt aus nicht-ionischen direktziehenden Farbstoffen,
(b) mindestens ein Alkylamidoalkylbetain in einer Gesamtmenge von 0,05 - 3 Gew.-%,
(c) mindestens ein nichtionisches Ethoxylat-Tensid, in einer Gesamtmenge von 0,2 - 1,5 Gew.-%, bevorzugt 0,4 - 1,2 Gew.-%, besonders bevorzugt 0,6 -0,9 Gew.-%,
(d) mindestens ein lineares 1-Alkanol mit 8 - 24 Kohlenstoffatomen in einer Gesamtmenge von 8 - 15 Gew.-%, bevorzugt 8 - 13 Gew.-% besonders bevorzugt 10 - 12 Gew.-%,
(e) mindestens ein wasserlösliches Magnesiumsalz in einer solchen Menge, dass der Gehalt an $Mg^{2+}$-Ionen 0,005 bis 1,000 Gew.-%, bevorzugt 0,008 bis 0,500 Gew.-%, besonders bevorzugt 0,010 bis 0,200 Gew.-%, besonders

bevorzugt 0,013 bis 0,100 Gew.-%, außerordentlich bevorzugt 0,018 bis 0,050 Gew.-%, beträgt,
(f) 74 - 86 Gew.-% Wasser,

wobei kein Alkylglycosid, kein Oxidationsfarbstoffvorprodukt und kein Oxidationsmittel enthalten sind,
wobei der Gesamttensidgehalt 0,5 - 6 Gew.-%, bevorzugt 1 - 5 Gew.-%, besonders bevorzugt 1,5 - 3 Gew.-% beträgt,
wobei sich alle Mengenangaben in Gew.-% auf das Gewicht des kosmetischen Mittels beziehen, dadurch gekennzeichnet, dass kein kationisches Tensid enthalten ist,
weiterhin dadurch gekennzeichnet, dass das Mittel einen pH-Wert im Bereich von 2,0 bis 5,5 aufweist, gemessen bei 22°C.

Unter keratinischen Fasern oder auch Keratinfasern sind dabei Pelze, Wolle, Federn und insbesondere menschliche Haare zu verstehen. Obwohl die Mittel in erster Linie zum Färben von Keratinfasern geeignet sind, steht prinzipiell einer Verwendung auch auf anderen Gebieten nichts entgegen.

Unter den verwendeten Begriffen "Mittel zum semipermanenten Färben von Haaren ", "Färbemittel" und "Tönungsmittel" werden Färbemittel verstanden, die die Haare auf Basis von direktziehenden Farbstoffen ohne den Einfluss eines Oxidationsmittels färben.

Als ersten wesentlichen Inhaltsstoff enthält das erfindungsgemäße Mittel mindestens einen direktziehenden Farbstoff (a), der aus nicht-ionischen direktziehenden Farbstoffen ausgewählt ist.

Der bzw. die direktziehenden Farbstoffe, einschließlich des mindestens einen nicht-ionischen direktziehenden Farbstoffs (a), ist bzw. sind bevorzugt in einer Gesamtmenge von 0,001 bis 7 Gew.-%, weiter bevorzugt von 0,01 bis 5,5 Gew.-%, weiter bevorzugt von 0,08 bis 3,4 Gew.-%, weiter bevorzugt von 0,1 bis 2 Gew.-%, weiter bevorzugt von 0,3 bis 1,5 Gew.-% und besonders bevorzugt von 0,6 bis 1 Gew.-%, jeweils bezogen auf das Gewicht des Färbemittels, enthalten.

Direktziehende Farbstoffe können allgemein in anionische, kationische und nicht-ionische direktziehende Farbstoffe unterteilt werden. Die direktziehenden Farbstoffe sind üblicherweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen.

Bei Farbstoffen, die ausschließlich anionische Ladungen tragen, spricht der Fachmann von Säurefarbstoffen. Die Begriffe anionischer Farbstoff und Säurefarbstoff werden im Sinne dieser Erfindung daher synonym verwendet. Unter anionischen Farbstoffen bzw. Säurefarbstoffen werden direktziehende Farbstoffe verstanden, die mindestens eine Carbonsäuregruppe (-COOH) und/oder mindestens eine Sulfonsäuregruppe ($-SO_3H$) besitzen. In Abhängigkeit vom pH-Wert liegen die protonierten Formen (-COOH, $-SO_3H$) der Carbonsäure- bzw. Sulfonsäuregruppen mit ihren deprotonierten Formen ($-COO^-$, $-SO_3^-$) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalenten an Kationen (wie beispielsweise Na-Kation oder K-Kationen) neutralisiert. Ein anionischer Farbstoff trägt keine kationischen Ladungen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403, CI 10316, COLIPA n° B001), Acid Yellow 3 (COLIPA n°:C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (CI 16230; Orange G sodium salt), Acid Orange 11 (CI 45370), Acid Orange 15 (CI 50120), Acid Orange 20 (CI 14600), Acid Orange 24 (BROWN 1; CI 20170; KATSU201; no sodium salt; Brown No.201; RESORCIN BROWN; ACID ORANGE 24; Japan Brown 201; D & C Brown No.1), Acid Red 14 (CI14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI CI18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (D&C Red; Red 104; AKA231; RED 28; SUREDYE; 11969 Red; PHLOXINE;CI 45405;CI 45410; EOSINE B); Acid Red 95 (CI 45425, Erythrosine, Simacid Erythrosine Y), Acid Red 184 (CI 15685), Acid Red 195; Pigment Red 57:1 (E180;D&CRED7; CI 15850; Rubine 4BN; CI 15850:1; PIGMENT RED 57; Litholrubine BK; LITHOLRUBINE RB; LITHOLRUBINE BCA; Lithol Rubine B); Acid Violet 43 (Jarocol Violet 43, Ext. D&C Violet n° 2, CI 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, CI Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (CI 42100), Acid Green 22 (CI 42170), Acid Green 25 (CI 61570, Japan Green 201, D&C Green No. 5), Acid Green 50 (Brillantsäuregrün BS, CI 44090, Acid Brilliant

Green BS, E 142), Acid Black 1 (Black n° 401, Naphthalene Black 10B, Amido Black 10B, CI 20470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Brown 1, Bromphenolblau und/oder Tetrabromphenolblau sowie Mischungen hiervon.

Der bzw. die anionische(n), direktziehende(n) Farbstoff(e) sind bevorzugt in einer Gesamtmenge von 0,001 bis 7 Gew.-%, bevorzugt von 0,01 bis 5,5 Gew.-%, weiter bevorzugt von 0,1 bis 3,4 Gew.-% und besonders bevorzugt von 0,3 bis 2 Gew.-%, jeweils bezogen auf das Gewicht des Färbemittels, enthalten.

Farbstoffe, die ausschließlich kationische Ladungen tragen, werden üblicherweise auch als basische Farbstoffe bezeichnet.

Als Beispiele für geeignete basische (kationische) Farbstoffe sind zu nennen: Basic Blue 6 (CI-No. 51,175), Basic Blue 7 (CI-No. 42,595) Basic Blue 9 (CI-No. 52,015), Basic Blue 26 (CI-No. 44,045), Basic Blue 41 (CI-No. 11,154), Basic Blue 99 (CI-No. 56,059), HC Blue 15, HC Blue 16 (Bluequat-Bromid), Cationic Blue 347, Basic Brown 4 (CI-No. 21,010), Basic Brown 16 (CI-No. 12,250), Basic Brown 17 (CI-No. 12,251), Natural Brown 7 (CI-No. 75,500), Basic Green 1 (CI-No. 42,040), Basic Red 2 (CI-No. 50,240), Basic Red 22 (CI-No. 11,055), Basic Red 51, Basic Red 76 (CI-No. 12,245), Basic Violet 1 (CI-No. 42,535), Basic Violet 2, Basic Violet 3 (CI-No. 42,555), Basic Violet 10 (CI-No. 45,170), Basic Violet 14 (CI-No. 42,510), Basic Yellow 57 (CI-No. 12,719), Basic Yellow 87 und Basic Orange 31, sowie Kombinationen der genannten Farbstoffe.

Als besonders geeignet haben sich ein oder mehrere Farbstoffe aus der Gruppe HC Blue 15, HC Blue 16 (Bluequat-Bromid), Cationic Blue 347, Basic Violet 2, Basic Red 51, Basic Red 76, Basic Yellow 57, Basic Yellow 87, Basic Orange 31, und Basic Brown 17 erwiesen.

Der bzw. die kationische(n), direktziehende(n) Farbstoff(e) sind bevorzugt in einer Gesamtmenge von 0,001 bis 7 Gew.-%, bevorzugt von 0,01 bis 5,5 Gew.-%, weiter bevorzugt von 0,1 bis 3,4 Gew.-% und besonders bevorzugt von 0,3 bis 2 Gew.-%, jeweils bezogen auf das Gewicht des Färbemittels, enthalten.

Die erfindungsgemäßen Färbemittel sind dadurch gekennzeichnet, dass sie als direktziehenden Farbstoff (a) mindestens einen nicht-ionischen direktziehenden Farbstoff enthalten. Dieser kann beispielsweise ausgewählt sein aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, HC Blue 15, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-ophenylendiamin, 6-Nitro-1,2,3,4-tetrahydrochinoxalin, 2-Hydroxy-1,4-naphthochinon, N,N'-bis-(2-Hydroxyethyl)-2-nitro-p-phenylendiamin, 2-Amino-6-chloro-4-nitrophenol, 4-Ethylamino-3-nitrobenzoesäure und 2-Chlor-6-ethylamino-4-nitrophenol.

Ganz besonders bevorzugt ist ein erfindungsgemäßes Mittel dadurch gekennzeichnet, dass es mindestens einen nicht-ionischen, direktziehenden Farbstoff (a) aus der Gruppe HC Red 1, HC Red 3, HC Red 7, HC Red 13, HC Orange 1, HC Yellow 2, HC Yellow 13, HC Blue 2, HC Blue 11, HC Blue 12, HC Blue 14, HC Violet 2, Disperse Violet 1, 4-[(3-Hydroxypropyl)amino]-3-nitrophenol und 4-Amino-3-nitrophenol umfasst.

Der bzw. die nicht-ionische(n), direktziehende(n) Farbstoff(e) (a) sind bevorzugt in einer Gesamtmenge von 0,001 bis 7 Gew.-%, bevorzugt von 0,01 bis 5,5 Gew.-%, weiter bevorzugt von 0,1 bis 3,4 Gew.-% und besonders bevorzugt von 0,3 bis 2 Gew.-%, jeweils bezogen auf das Gewicht des Färbemittels, enthalten.

Als bevorzugte direktziehende Farbstoffe sind hervorzuheben: Basic Red 76, HC Blue 16, Basic Yellow 57, 4-Hydroxypropylamino-3-nitrophenol, HC Red 13, HC Red 3, HC Blue 12, HC Orange 1, HC Blue 16 (Bluequat-Bromid), HC Yellow 2, N,N'-bis-(2-Hydroxyethyl)-2-nitro-p-phenylendiamin, 4-Amino-3-nitrophenol, Ext. D&C Violet 2, HC Violet 2, Basic Brown 17, Basic Red 76 und Basic Yellow 57, sowie Gemische davon. Stärker bevorzugt sind Basic Red 76, HC Blue 16, Basic Yellow 57, 4-Hydroxypropylamino-3-nitrophenol, 4-Amino-3-nitrophenol, HC Red 13, HC Violet 2, sowie Gemische davon. Besonders bevorzugt sind HC Blue 16, Basic Violet 2, Basic Yellow 57, 4-Hydroxypropylamino-3-nitrophenol, HC Red 13 und 4-Amino-3-nitrophenol sowie Gemische davon. In einer bevorzugten Ausführungsform umfasst das erfindungsgemäße Mittel eine Kombination mehrerer direktziehender Farbstoffe. Durch Kombination einer Vielzahl direktziehender Farbstoffe können Nuancen im gesamten Farbspektrum erzeugt werden. Dabei können sowohl mehrere rein nicht-ionische Farbstoffe miteinander kombiniert werden als auch Kombination von anionischen, kationischen und/oder nicht-ionischen Farbstoffe miteinander eingesetzt werden.

Als weiteren obligatorischen Inhaltsstoff enthalten die erfindungsgemäßen Mittel mindestens ein Alkylamidoalkylbetain in einer Gesamtmenge von 0,05 - 3 Gew.-%, bevorzugt 0,1 - 1,5 Gew.-%, besonders bevorzugt 0,5 - 1,0 Gew.-%, bezogen auf das Gewicht des Mittels.

Alkylamidoalkylbetaine fungieren als amphoteres Tensid.

In einer bevorzugten Ausführungsform ist das mindestens eine Alkylamidoalkylbetain ausgewählt aus mindestens einem Alkylamidoalkylbetain der folgenden Formel (I):

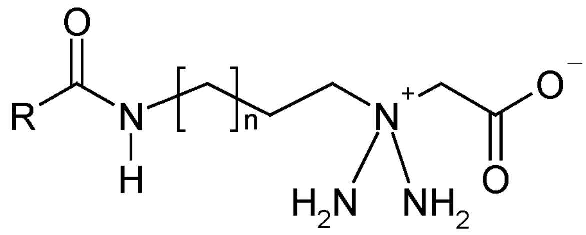

(I)

wobei

R einen linearen oder verzweigten, gesättigten oder ungesättigten Kohlenwasserstoffrest mit 7 bis 21, bevorzugt 9 bis 17 Kohlenstoffatomen, besonders bevorzugt 11 bis 13 darstellt, und
n eine ganze Zahl von 0 bis 4, insbesondere 1 bis 2 ist.

In einer bevorzugten Ausführungsform ist R ein linearer Kohlenwasserstoffrest, insbesondere ein linearer, gesättigter Kohlenwasserstoffrest.

In einer ganz besonders bevorzugten Ausführungsform wird ein Alkylamidoalkylbetain der Formel (I) mit R = $-C_9H_{19}$, $-C_{11}H_{23}$ oder $-C_{13}H_{27}$ und n = 0, 1 oder 2 eingesetzt, insbesondere ein Alkylamidoalkylbetain der Formel (I) mit R = $-C_{11}H_{23}$ und n = 1 als Alkylamidoalkylbetain (b) eingesetzt, auch bekannt als Lauramidopropylbetain.

In einer außerordentlich bevorzugten Ausführungsform wird ein Alkylamidoalkylbetain der Formel (I) mit n = 0, 1 oder 2 eingesetzt, wobei der Rest RCO von Kokosfettsäuren abstammt, also eine Mischung von n-Decanoyl bis n-Stearoyl darstellt mit einem wesentlichen Anteil an n-Lauroyl.

Dieses amphotere Tensid ist auch bekannt als Cocamidopropylbetain.

Übliche Alkylamidoalkylbetain-Rohstoffe enthalten allerdings, bedingt durch den Herstellprozess, einen beträchtlichen Gehalt an Natriumchlorid, der 4,5 bis 6 Gew.-%, bezogen auf die Alkylamidoalkylbetain-Lösung, betragen kann. Überraschend wurde festgestellt, dass der Einsatz einer Natriumchlorid-armen Alkylamidoalkylbetain-Lösung, insbesondere einer Natriumchlorid-armen Alkylamidopropylbetain-Lösung, die Färbeeigenschaften weiter verbessern kann. Erfindungsgemäß bevorzugte Mittel enthalten daher, jeweils bezogen auf ihr Gewicht, Natriumchlorid in einer Menge von 0 bis 0,060 Gew.-%, bevorzugt 0,005 - 0,040 Gew.-%, besonders bevorzugt 0,010 - 0,030 Gew.-%. Vorzugsweise ist die wässrige Handelsprodukt-Zubereitung des Alkylamidoalkylbetains (b) im Wesentlichen frei von Natriumchlorid, das heißt, sie umfasst maximal 0,6 Gew.-%, bevorzugt maximal 0,5 Gew.-%, besonders bevorzugt < 0,2 Gew.-% Natriumchlorid, jeweils bezogen auf das Gesamtgewicht der wässrigen Handelsprodukt-Zubereitung des Alkylamidoalkylbetains (b). Weitere erfindungsgemäß bevorzugte Mittel sind dadurch gekennzeichnet, dass der Gehalt an Natriumchlorid 0 bis 0,060 Gew.-%, bevorzugt 0,005 - 0,040 Gew.-%, besonders bevorzugt 0,010 - 0,030 Gew.-%, jeweils bezogen auf das Gewicht des erfindungsgemäßen Mittels beträgt.

Als weiteren obligatorischen Inhaltsstoff enthalten die erfindungsgemäßen Mittel mindestens ein nichtionisches Ethoxylat-Tensid in einer Gesamtmenge von 0,2 - 1,5 Gew.-%, bevorzugt 0,4 - 1,2 Gew.-%, besonders bevorzugt 0,6 - 0,9 Gew.-%, bezogen auf das Gewicht des Mittels.

Bevorzugte nichtionische Ethoxylat-Tensid sind ausgewählt aus ethoxylierten $C_8$-$C_{24}$-Alkanolen mit 10 - 50 Mol Ethylenoxid pro Mol, ethoxylierten $C_8$-$C_{24}$-Carbonsäuren mit 10 - 50 Mol Ethylenoxid pro Mol und ethoxylierten Triglyceriden mit 10 - 50 Mol Ethylenoxid pro Mol, insbesondere ethoxyliertem Ricinusöl, das optional gehärtet bzw. hydriert sein kann, sowie Mischungen hiervon.

Die ethoxylierten $C_8$-$C_{24}$-Alkanole haben die Formel $R^1O(CH_2CH_2O)_nH$, wobei $R^1$ steht für einen linearen oder verzweigten Alkyl- und/oder Alkenylrest mit 8 - 24 Kohlenstoffatomen und n, die Anzahl der Ethylenoxid-Einheiten pro Molekül, für Zahlen von 10 - 50, vorzugsweise 12 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 50 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten $C_8$-$C_{24}$-Carbonsäuren haben die Formel $R^1(OCH_2CH_2)_nOH$, wobei $R^1$ steht für einen linearen oder verzweigten gesättigten oder ungesättigten Acylrest mit 8 -24 Kohlenstoffatomen und n, die Anzahl der Ethylenoxid-Einheiten pro Molekül, für Zahlen von 10 - 50, bevorzugt 12 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 50 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-50-monooleat, und PEG-50-monolaurat sowie Mischungen hiervon.

Besonders bevorzugt enthalten sind die $C_{12}$-$C_{18}$-Alkanole oder die $C_{12}$-$C_{18}$-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20 sowie Mischungen hiervon, insbesondere die Mischung aus Ceteth-20 und Steareth-20, die auch als Ceteareth-20 bezeichnet wird.

Weiterhin bevorzugt sind ethoxylierte Triglyceride mit 10 - 50 Mol Ethylenoxid pro Mol, insbesondere ethoxyliertes Ricinusöl mit 10 - 50 Mol Ethylenoxid pro Mol, das optional gehärtet bzw. hydriert sein kann, bevorzugt Verbindungen mit den INCI-Bezeichnungen PEG-40 Castor Oil und PEG-40 Hydrogenated Castor Oil.

Als weiteren obligatorischen Inhaltsstoff enthalten die erfindungsgemäßen Mittel mindestens ein lineares 1-Alkanol mit 8 - 24 Kohlenstoffatomen in einer Gesamtmenge von 8 - 15 Gew.-%, bevorzugt 8 - 13 Gew.-% besonders bevorzugt 10 - 12 Gew.-%, bezogen auf das Gewicht des Mittels. Bevorzugte lineare 1-Alkanole mit 8 - 24 Kohlenstoffatomen sind ausgewählt aus Laurylalkohol, Cetylalkohol, Stearylalkohol, Arachidylalkohol, Behenylalkohol und Lanolinalkohol sowie Mischungen hiervon, sowie auch ausgewählt aus Gemischen dieser Alkohole, wie sie bei der technischen Hydrierung von pflanzlichen und tierischen Fettsäuren erhältlich sind, z. B. Kokosalkohol. Außerordentlich bevorzugt sind Cetylalkohol, Stearylalkohol und Kokosalkohol sowie Mischungen hiervon.

Als weiteren obligatorischen Inhaltsstoff enthalten die erfindungsgemäßen Mittel mindestens ein wasserlösliches Magnesiumsalz in einer solchen Menge, dass der Gehalt an $Mg^{2+}$-Ionen 0,005 bis 1,000 Gew.-%, bevorzugt 0,008 bis 0,500 Gew.-%, besonders bevorzugt 0,010 bis 0,200 Gew.-%, besonders bevorzugt 0,013 bis 0,100 Gew.-%, außerordentlich bevorzugt 0,018 bis 0,050 Gew.-%, beträgt, jeweils bezogen auf das Gewicht des erfindungsgemäßen Mittels.

Der Gehalt an wasserlöslichem Magnesiumsalz, wie beispielsweise Magnesiumchlorid oder Trimagnesiumdicitrat, bewirkte überraschend eine verbesserte Waschechtheit sowie zumindest teilweise eine verbesserte Chromatizität gegenüber der gleichen Rezeptur ohne wasserlösliches Magnesiumsalz.

Unter einem wasserlöslichen Magnesiumsalz wird erfindungsgemäß ein Magnesiumsalz mit einer Wasserlöslichkeit von mindestens 5 Gramm pro Liter Lösung bei 20°C verstanden. Das erfindungsgemäß besonders bevorzugte Magnesiumchlorid hat eine Wasserlöslichkeit von 542 g/Liter Lösung bei 20 °C. Eine Verbindung wie Magnesiumcarbonat mit einer Wasserlöslichkeit von lediglich 0,106 Gramm pro Liter Lösung bei 20°C ist daher kein wasserlösliches Magnesiumsalz im Sinne der vorliegenden Erfindung.

Erfindungsgemäß bevorzugte wasserlösliche Magnesiumsalze sind ausgewählt aus Magnesiumchlorid (95,21 g/mol, wasserfrei), Magnesiumsulfat, Magnesiumbromid, Magnesiumbenzoat, Magnesiumformiat, Magnesiumacetat, Magnesiumpropionat, Magnesiumnitrat, Trimagnesiumdicitrat (451,13 g/mol, wasserfrei) und Magnesiumgluconat sowie Mischungen dieser Salze.

Weitere erfindungsgemäß bevorzugte wasserlösliche Magnesiumsalze sind ausgewählt aus wasserlöslichen Magnesiumsalzen mit einem nicht-chelatisierenden Anion, besonders bevorzugt ausgewählt aus Magnesiumchlorid, Magnesiumsulfat, Magnesiumbromid, Magnesiumbenzoat, Magnesiumformiat, Magnesiumacetat, Magnesiumpropionat und Magnesiumnitrat sowie Mischungen dieser Salze. Erfindungsgemäß außerordentlich bevorzugt sind Magnesiumchlorid und Magnesiumsulfat sowie Mischungen hiervon; höchst bevorzugt ist Magnesiumchlorid.

Die erfindungsgemäßen Mittel enthalten weiterhin 74 - 86 Gew.-%, bevorzugt 78 - 84 Gew.-% Wasser, jeweils bezogen auf das Gewicht des Mittels.

Der Gesamttensidgehalt der erfindungsgemäßen Mittel beträgt 0,5 - 6 Gew.-%, bevorzugt 1 - 5 Gew.-%, besonders bevorzugt 1,5 - 3 Gew.-%, jeweils bezogen auf das Gewicht des Mittels. Dies unterstützt die Haarkonditionierende Wirkung des erfindungsgemäßen Mittels.

Weiterhin sind die erfindungsgemäßen Mittel dadurch charakterisiert, dass kein Alkylglycosid, kein Oxidationsfarbstoffvorprodukt und kein Oxidationsmittel enthalten sind.

Die erfindungsgemäß ausgeschlossenen Alkylglycoside sind nicht-ionische Tenside, die sich durch die Abwesenheit elektrischer Ladungen in den Molekülen auszeichnen. Erfindungsgemäß ausgeschlossene Alkylpolyglycoside zeichnen sich dadurch aus, dass sie einen hydrophoben, langkettigen Alkylrest und als hydrophilen Molekülteil einen Glycosidzucker aufweisen, beispielsweise Laurylpolyglucosid, Decylpolyglucosid, Octylpolyglucosid und Kokosglucosid (Cocoglucosid).

Unter den Begriffen "Tenside" und "Emulgatoren" werden grenzflächenaktive Substanzen verstanden, die an Ober- und Grenzflächen Adsorptionsschichten bilden oder in Volumenphasen zu Mizellkolloiden oder lyotropen Mesophasen aggregieren können. Basiseigenschaften der Tenside und Emulgatoren sind die orientierte Absorption an Grenzflächen sowie die Aggregation zu Mizellen und die Ausbildung von lyotropen Phasen.

Die erfindungsgemäßen Mittel enthalten kein kationisches Tensid. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren an Grenzflächen und aggregieren in wässriger Lösung oberhalb der kritischen Mizellbildungskonzentration zu positiv geladenen Mizellen.

Beispiele für erfindungsgemäß ausgeschlossene Kationtenside sind:

- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen, oder
- tertiäre Sulfonium-Salze.

Weiterhin kann die kationische Ladung auch in Form einer Onium-Struktur Bestandteil eines heterozyklischen Ringes (z.B. eines Imidazoliumringe odereiner Pyridiniumringes) sein. Neben der funktionellen Einheit, welche die kationische Ladung trägt, kann das Kationtensid auch weitere ungeladene funktionelle Gruppen beinhalten, wie es beispielsweise bei Esterquats der Fall ist. Kationische Tenside dieses Typs sind beispielsweise Salze des N,N,N-Trimethyl-1-hexadecan-aminiums, insbesondere N,N,N-Trimethyl-1-hexadecanaminiumchlorid, Salze des Dimethyldistearyldimethylammoniums und kationische Imidazoliumverbindungen.

In einer bevorzugten Ausführungsform enthalten die erfindungsgemäßen Mittel kein anionisches Tensid. Bei anionischen Tensiden umfasst der hydrophile Molekülteil eine negativ geladene hydrophile Kopfgruppe. Bei der negativ geladenen hydrophilen Kopfgruppe kann es sich beispielsweise um eine Carbonsäuregruppe bzw. das Salz einer Carbonsäuregruppe, eine Sulfonsäuregruppe bzw. das Salz der Sulfonsäuregruppe, eine Schwefelsäureestergruppierung bzw. das Salz hiervon, eine Phosphonsäuregruppe bzw. das Salz der Phosphonsäuregruppe, oder eine Phosphorsäureestergruppierung bzw. das Salz hiervon handeln.

Anionische Tenside sind demnach gekennzeichnet durch die Anwesenheit einer wasserlöslich machenden, anionischen Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Typische Beispiele für anionische Tenside sind Alkylbenzolsulfonate, Alkansulfonate, Olefinsulfonate, Alkylethersulfonate, Glycerinethersulfonate, alpha-Methylestersulfonate, Sulfofettsäuren, Alkylsulfate, Fettalkoholethersulfate, Glycerinethersulfate, Hydroxymischethersulfate, Monoglycerid(ether) sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretaurate, Acyllactylate, Acyltartrate, Acylglutamate, Acylaspartate, Alkyloligoglucosidsulfate, Proteinfettsäurekondensate und Alkyl-(ether)phosphate.

In einer weiteren, besonders bevorzugten Ausführungsform umfasst das erfindungsgemäße Mittel nur geringe Mengen an anionischen Tensiden. Vorzugsweise umfasst das erfindungsgemäße Mittel anionische Tenside in einer Gesamtmenge von 0 bis ≤ 0,2 Gew.-%, weiter bevorzugt von 0 bis ≤ 0,15 Gew.-%, und insbesondere von 0 bis ≤ 0,1 Gew.-%, bezogen auf das Gesamtgewicht des Mittels. Ganz besonders bevorzugt das Mittel frei von anionischen Tensiden.

In einer weiteren, besonders bevorzugten Ausführungsform umfasst das erfindungsgemäße Mittel als Tenside ausschließlich das mindestens eine Alkylamidoalkylbetain (b) in Kombination mit dem mindestens einen nichtionischen Ethoxylat-Tensid (c) in den anspruchsgemäßen Mengen.

Die erfindungsgemäßen Färbemittel können weiterhin zusätzliche Wirk-, Hilfs- und Zusatzstoffe enthalten, um die Färbeleistung zu verbessern und weitere gewünschte Eigenschaften der Mittel einzustellen.

Färbeprozesse auf Keratinfasern laufen üblicherweise im mittleren sauren bis alkalischen Bereich, bevorzugt im schwach sauren bis schwach alkalischen Milieu ab. Der pH-Wert des erfindungsgemäßen Mittels liegt im Bereich von pH 2,0 bis pH 5,5. Um die Keratinfasern so weit wie möglich zu schonen und insbesondere, um eine besonders deutliche Haarkonditionierende Wirkung zu erzielen, weisen die erfindungsgemäßen Färbemittel einen pH-Wert im Bereich von 2,0 bis 5,5, bevorzugt 3,5 bis pH 5,5, besonders bevorzugt von 4,0 bis 5,5, weiter bevorzugt von 4,1 bis 5,0 und ganz besonders bevorzugt von 4,3 bis 4,8, auf, jeweils gemessen bei 22°C.

Die Messung des pH-Wertes kann beispielsweise mit einer Glaselektrode erfolgen, die üblicherweise in Form einer Einstabmesskette ausgeführt ist. Bei den pH-Werten der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkali-metallmetasilikaten, (Erd-)Alkalimetallphosphaten und (Erd-)Alkalimetallhydrogenphosphaten, ausgewählt werden. Bevorzugte anorganische Alkalisierungsmittel sind Natriumhydroxid und Kaliumhydroxid sowie Mischungen hiervon. Erfindungsgemäß einsetzbare, organische Alkalisierungsmittel sind bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im

Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Zur Einstellung des pH-Werts verwendbare Ansäuerungsmittel sind organische Säuren, wie Citronensäure, Essigsäure, Ascorbinsäure, Benzoesäure, Milchsäure, Apfelsäure und Maleinsäure, sowie mineralische Säuren, wie Salzsäure, Schwefelsäure oder Phosphorsäure.

Weiterhin hat es sich als vorteilhaft erweisen, wenn die Färbemittel mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltige Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/ oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nicht-ionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/ oder Hydroxylgruppen (Dimethiconcopolyole), lineare PolysiloxanA)-PolyoxyalkylenB)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie Polyacrylsäuren oder vernetzte Polyacrylsäuren; haarkonditionierende Verbindungen wie Phospholipide, beispielsweise Lecitin und Kephaline; Parfümöle, Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; Lichtschutzmittel und UV-Blocker; Wirkstoffe wie Panthenol, Pantothensäure, Pantolacton, Allantoin, Pyrrolidoncarbonsäuren und deren Salze sowie Bisabolol; Ceramide oder Pseudoceramide; Vitamine, Provitamine und Vitaminvorstufen; Pflanzenextrakte.

Die Auswahl dieser weiteren Stoffe wird der Fachmann gemäß den gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe sind in den erfindungsgemäßen Mitteln bevorzugt in Mengen von jeweils 0,0001 bis 2 Gew.-%, insbesondere von 0,01 bis 1,5 Gew.-%, bezogen auf das Gesamtgewicht der jeweiligen Mittel, enthalten.

Die erfindungsgemäßen Mittel können zusätzlich weitere organische Lösemittel, wie beispielsweise einen $C_1$-$C_4$-Alkohol, insbesondere Ethanol bzw. Isopropanol, weiterhin 4-Methoxybutanol, Ethyldiglykol, 1,2-Propylenglykol, n-Propanol, n-Butanol, 1,3-Butylenglykol, Glycerin, Diethylenglykolmonoethylether, und Diethylenglykolmono-n-butylether, enthalten. Bevorzugt sind dabei alle wasserlöslichen organischen Lösemittel. Bevorzugte erfindungsgemäße Mittel sind dadurch gekennzeichnet, dass sie zusätzlich ein nichtwässriges Lösemittel enthalten, wobei bevorzugte erfindungsgemäße Mittel das Lösemittel in einer Konzentration von 0,1 bis 8 Gew.-%, bevorzugt in einer Konzentration von 0,5 bis 5 Gew.-%, ganz besonders bevorzugt in einer Konzentration von 1 bis 3 Gew.-%, jeweils bezogen auf das Gesamtgewicht des Mittels, enthalten.

Darüber hinaus kann auch noch mindestens einen Penetrationsverstärker enthalten sein. Penetrationsverstärker können in der Regel auch als Lösemittel fungieren können.

Beispielhaft für geeignete Penetrationsverstärker können hierbei Propylencarbonat, Benzylalkohol, 2-Phenoxyethan-1-ol und/oder Benzylalkohol genannt werden.

In einer weiteren ganz besonders bevorzugten Ausführungsform ist ein erfindungsgemäßes Mittel deshalb dadurch gekennzeichnet, dass es zusätzlich mindestens einen Penetrationsverstärker aus der Gruppe aus Propylencarbonat, Benzylalkohol, 2-Phenoxyethan-1-ol und/oder Benzylalkohol enthält.

Weiterhin kann das erfindungsgemäße Mittel ein oder mehrere kationische Polymere, ausgewählt aus Polyquaternium-1, Polyquaternium-2, Polyquaternium-3, Polyquaternium-4, Polyquaternium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquaternium-64, Polyquaternium-67, Polyquaternium-68, Polyquaternium-69 und Polyquaternium-86 sowie Mischungen hiervon zur Verbesserung der konditionierenden

Wirkung des Mittels wie auch zur Verbesserung der Färbeeigenschaften enthalten.

Als besonders bevorzugtes kationisches Polymer ist Polyquaternium-6 hervorzuheben. Dies verbessert die Eigenschaften des Mittels und die Qualität der erhaltenen Färbung.

Bevorzugt ist mindestens eine kationische Polyquaternium-Verbindung in einer Gesamtmenge von 0,005 bis 1 Gew.-%, besonders bevorzugt 0,01 bis 0,8 Gew.-%, stärker bevorzugt 0,05 bis 0,5 Gew.-% und insbesondere in einer Menge von 0,1 bis 0,2 Gew.-%, jeweils bezogen auf das Gewicht des Mittels, enthalten.

Besonders bevorzugt ist Polyquaternium-6 in einer Gesamtmenge von 0,005 bis 1 Gew.-%, besonders bevorzugt 0,01 bis 0,8 Gew.-%, stärker bevorzugt 0,05 bis 0,5 Gew.-% und insbesondere in einer Menge von 0,1 bis 0,2 Gew.-%, jeweils bezogen auf das Gewicht des Mittels, enthalten.

Weiterhin kann bevorzugt mindestens ein Silicon in den erfindungsgemäßen Mitteln enthalten sein, um die haarkonditionierende Wirkung weiter zu verbessern. Ein besonders bevorzugtes Silicon ist Polydimethylsiloxan mit der INCI Dimethicone. Erfindungsgemäß geeignete Polydimethylsiloxane und Dimethicone sind sowohl solche mit einem niedrigen Polymerisationsgrad von 2 bis 20 als auch solche mit einem mittleren Polymerisationsgrad von 21 bis 1000 als auch solche mit einem Polymerisationsgrad von mehr als 1000, insbesondere solche mit einem hohen Polymerisationsgrad von mehr als 1000 bis 25.000. Die hoch polymerisierten Polydimethylsiloxane und Dimethicone weisen auch hohe Viskositäten auf und liegen eher als Silicongum denn als Öl vor. Derartig hoch polymerisierte Polydimethylsiloxane und Dimethicone mit einem Polymerisationsgrad von mehr als 1000 bis 25.000 werden erfindungsgemäß bevorzugt in voremulgierter Form eingesetzt, besonders bevorzugt mit einer zahlenmittleren Tröpfchengröße bzw. Partikelgröße von 0,05 $\mu$m bis 20 $\mu$m, bevorzugt 0,5 $\mu$m bis 10 $\mu$m.

Weiterhin kann bevorzugt mindestens ein pflanzliches Öl in den erfindungsgemäßen Mitteln enthalten sein, um die haarkonditionierende Wirkung weiter zu verbessern. Besonders bevorzugte pflanzliche Öle sind ausgewählt aus Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkern-öl, Grapefruitsamenöl, Hanföl, Haselnussöl, Holundersamenöl, Johannisbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl und den flüssigen Anteile des Kokosöls sowie Mischungen der vorgenannten Öle.

Bevorzugt ist mindestens ein pflanzliches Öl in einer Gesamtmenge von 0,005 bis 1 Gew.-%, besonders bevorzugt 0,01 bis 0,8 Gew.-%, stärker bevorzugt 0,05 bis 0,5 Gew.-% und insbesondere in einer Menge von 0,1 bis 0,2 Gew.-%, jeweils bezogen auf das Gewicht des Mittels, enthalten.

Besonders bevorzugt ist Marulaöl in einer Gesamtmenge von 0,005 bis 1 Gew.-%, besonders bevorzugt 0,01 bis 0,8 Gew.-%, stärker bevorzugt 0,05 bis 0,5 Gew.-% und insbesondere in einer Menge von 0,1 bis 0,2 Gew.-%, jeweils bezogen auf das Gewicht des Mittels, enthalten.

Der erfindungsgemäße Conditioner kann unmittelbar auf die zu färbenden keratinischen Fasern aufgebracht werden und der Applikationsvorgang kann problemlos in die alltägliche Hygiene des Anwenders integriert werden.

Ein weiterer Gegenstand der vorliegenden Erfindung ist daher ein Verfahren zum Färben menschlicher Haare, bei dem ein kosmetisches Mittel zum gleichzeitigen semipermanenten Färben und Pflegen von Haaren, enthaltend

(a) mindestens einen direktziehenden Farbstoff, ausgewählt aus nicht-ionischen direktziehenden Farbstoffen,
(b) mindestens ein Alkylamidoalkylbetain in einer Gesamtmenge von 0,05 - 3 Gew.-%,
(c) mindestens ein nichtionisches Ethoxylat-Tensid, in einer Gesamtmenge von 0,2 - 1,5 Gew.-%, bevorzugt 0,4 - 1,2 Gew.-%, besonders bevorzugt 0,6 -0,9 Gew.-%,
(d) mindestens ein lineares 1-Alkanol mit 8 - 24 Kohlenstoffatomen in einer Gesamtmenge von 8 - 15 Gew.-%, bevorzugt 8 - 13 Gew.-% besonders bevorzugt 10 - 12 Gew.-%,
(e) mindestens ein wasserlösliches Magnesiumsalz in einer solchen Menge, dass der Gehalt an $Mg^{2+}$-Ionen 0,005 bis 1,000 Gew.-%, bevorzugt 0,008 bis 0,500 Gew.-%, besonders bevorzugt 0,010 bis 0,200 Gew.-%, besonders bevorzugt 0,013 bis 0,100 Gew.-%, außerordentlich bevorzugt 0,018 bis 0,050 Gew.-%, beträgt,
(f) 74 - 86 Gew.-% Wasser,

wobei kein Alkylglycosid, kein Oxidationsfarbstoffvorprodukt und kein Oxidationsmittel enthalten sind,
wobei der Gesamttensidgehalt 0,5 - 6 Gew.-%, bevorzugt 1 - 5 Gew.-%, besonders bevorzugt 1,5 - 3 Gew.-% beträgt,
wobei sich alle Mengenangaben in Gew.-% auf das Gewicht des kosmetischen Mittels beziehen,
wobei kein kationisches Tensid enthalten ist und das Mittel einen pH-Wert im Bereich von 2,0 bis 5,5 aufweist, gemessen bei 22°C,
auf die Haare appliziert und nach einer Einwirkzeit von 1 Minute bis 30 Minuten, bevorzugt 5 bis 20 Minuten bei 20 - 50 °C, bevorzugt 20 - 32 °C, ausgespült wird, optional gefolgt vom Trocknen der Haare.

Bezüglich weiterer bevorzugter Ausführungsformen des erfindungsgemäßen Verfahrens gilt *mutatis mutandis*

das zu den erfindungsgemäßen Mitteln Gesagte.

Beispiele

Die nachstehenden Beispiele sollen den Gegenstand der Erfindung näher erläutern.

Versuchsreihe mit Nuance 6-88

Folgende Tönungsconditioner wurden hergestellt. E1 und E2 sind erfindungsgemäße Mittel, V1 und V2 dienen zum Vergleich. Alle Mengenangaben beziehen sich auf den Aktivsubstanzgehalt und sind in Gew.-%.

| | E1 | E2 | V1 | V2 |
|---|---|---|---|---|
| Coconut Alcohol | **4,9** | 4,9 | 4,9 | 4,9 |
| Cetearyl Alcohol | **9,7** | 9,7 | 9,7 | 9,7 |
| Propylparaben + Methylparaben | **0,6** | 0,6 | 0,6 | 0,6 |
| Cocamidopropyl Betaine | **0,7** | 0,7 | 0,7 | 0,7 |
| Ceteareth-20 | **0,9** | 0,9 | 0,9 | 0,9 |
| Monoethanolamin | **0,07** | 0,07 | 0,07 | 0,07 |
| Polyquaternium-6 | **0,2** | 0,2 | 0,2 | 0,2 |
| Etidronsäure | **0,05** | 0,05 | 0,05 | 0,05 |
| Citronensäure | **0,08** | 0,08 | 0,08 | 0,08 |
| Magnesiumchlorid (wasserfrei) | **0,1*** | - | - | - |
| Trimagnesiumdicitrat (wasserfrei) | **-** | 0,2** | - | - |
| Natriumchlorid | **0,01** | 0,01 | 0,01 | 0,12 |
| Marulaöl | **0,2** | 0,2 | 0,2 | 0,2 |
| Keratinhydrolysat mit Mw 400-800 Da | **0,02** | 0,02 | 0,02 | 0,02 |
| D-Panthenol | **0,2** | 0,2 | 0,2 | 0,2 |
| Cocodimonium Hydroxypropyl Hydrolyzed Keratin | **0,015** | 0,015 | 0,015 | 0,015 |
| Dimethicone*** | **0,5** | 0,5 | 0,5 | 0,5 |
| Laureth-4 | **0,03** | 0,03 | 0,03 | 0,03 |
| Laureth-23 | **0,03** | 0,03 | 0,03 | 0,03 |
| Parfum | **0,7** | 0,7 | 0,7 | 0,7 |
| Butylenglycol | **3,5** | 3,5 | 3,5 | 3,5 |
| HC Red 3 | **0,15** | 0,15 | 0,15 | 0,15 |
| 4-Hydroxypropylamino-3-Nitrophenol | **0,95** | 0,95 | 0,95 | 0,95 |
| Wasser @ 100,00 | **76,395** | 76,295 | 76,495 | 76,385 |

* entspricht 0,026 Gew.-% $Mg^{2+}$-Ionen im Tönungsconditioner
** entspricht 0,032 Gew.-% $Mg^{2+}$-Ionen im Tönungsconditioner
*** ex Xiameter MEM-2664 Emulsion

Alle farbmetrischen Messungen wurden mit dem Farbmetrik-Gerät Spectralflash SF 450 von Datacolor durchgeführt.

Für die Waschechtheitstests wurden Haarsträhnen vom Typ Kerling 11-0 verwendet.

Diese Haarsträhnen wurden jeweils mit den oben dargestellten Färbeformulierungen E1, E2, V1 bzw. V2 bei 20°C mit 10 Minuten Einwirkzeit gefärbt. Anschließend wurden die Haarsträhnen gründlich mit Wasser ausgespült und im Luftstrom getrocknet. Nach dem Färben und Trocknen wurden die gefärbten Haarsträhnen farbmetrisch vermessen. Die CIELAB-Werte für die gefärbten, ungewaschenen Strähnen sowie die daraus berechneten Werte C (Chroma) und h

(Farbton, "hue") sind in den Zeilen "E1", "E2", "V1" und "V2" angeführt.

Anschließend wurden die gefärbten Haarsträhnen zwölfmal mit einem handelsüblichen Shampoo (Schauma 7-Kräuter Shampoo) gewaschen. Jeweils nach 6, 10 und 12 Shampoo-Wäschen wurden die Haarsträhnen im Luftstrom getrocknet und farbmetrisch vermessen.

Der Farbabstand (ΔE) zwischen gefärbter, unshampoonierter Strähne und gefärbter, shampoonierter Strähne wurde gemäß der nachfolgenden Formel berechnet:

$$\Delta E = \sqrt{(Lv - Ln)^2 + (av - an)^2 + (bv - bn)^2},$$

mit

*Lv, av,* bv: Farbmetrikwerte vor der Shampoo-Wäsche

*Ln, an,* bn: Farbmetrikwerte nach 6, 10 oder 12 Shampoo-Wäschen

Die Chromatizität (Chroma c, Farbrichtung) wird aus den Messgrößen a (CIE) und b (CIE) gemäß folgender Formel berechnet:

$$c = \sqrt{a^2 + b^2}.$$

Die Messgröße ΔE, der so genannte Farbabstand, beschreibt die Waschechtheit der Färbung. Je größer der Farbabstand ΔE zwischen gefärbter, unshampoonierter Strähne und gefärbter, shampoonierter Strähne ist, desto stärker ist der durch die Shampoo-Wäsche resultierende Farbabzug bzw. das Auswaschen der Färbung. Die Waschechtheit ist folglich umso besser, je niedriger der Wert des Farbabstand ΔE ist.

Farbabstände von ΔE < 1 sind für das menschliche Auge nicht wahrnehmbar. Farbabstände von ΔE < 2 sind lediglich für das geschulte Auge sichtbar. Farbabstände von ΔE > 2 sind auch für das ungeschulte Auge sichtbar.

Die erfindungsgemäßen Färbemittel E1 und E2 unterscheiden sich von dem Vergleichsfärbemittel V1 nur durch den Zusatz an Magnesiumsalz in einer anspruchsgemäßen Menge. Man sieht deutlich, dass die CIE DE (ΔE) - Werte für E1 und E2 signifikant geringer sind als für V1 und V2, dass also der Zusatz an einer anspruchsgemäßen Menge eines wasserlöslichen Magnesiumsalzes die Waschechtheit signifikant verbessert.

Die erfindungsgemäßen Färbemittel E3 und E4 unterscheiden sich von dem Vergleichsfärbemittel V3 nur durch den Zusatz an Magnesiumsalz in einer anspruchsgemäßen Menge. Man sieht deutlich, dass die CIE DE (ΔE) - Werte für E3 und E4 signifikant geringer sind als für V3 und V4, dass also der Zusatz an einer anspruchsgemäßen Menge eines wasserlöslichen Magnesiumsalzes die Waschechtheit signifikant verbessert. Auch die Farbintensität ist in beiden Testreihen verbessert; dies ist zu erkennen an der Messgröße L, die möglichst niedrige Werte annehmen sollte.

Die farbmetrischen Daten für die Versuche mit der Nuance 6-88 sind in der folgenden Tabelle zusammengestellt:

| Std.-Name | Std. CIE L | Std. CIE a | Std. CIE b | Std. CIE C | Std. CIE h | |
|---|---|---|---|---|---|---|
| **E1** | 31,18 | 35,09 | 18,74 | 39,77 | 28,10 | |
| Batch Name | CIE L Muster | CIE a Muster | CIE b Muster | CIE C Muster | CIE h Muster | CIE DE (ΔE) |
| E1 nach 6 Wäschen | 42,02 | 34,68 | 19,78 | 39,93 | 29,70 | 10,90 |
| E1 nach 10 Wäschen | 46,19 | 33,23 | 18,89 | 38,22 | 29,61 | 15,13 |
| E1 nach 12 Wäschen | 46,61 | 30,50 | 18,10 | 35,47 | 30,68 | 16,12 |
| | | | | | | |
| Std.-Name | Std. CIE L | Std. CIE a | Std. CIE b | Std. CIE C | Std. CIE h | |
| **E2** | 31,70 | 36,66 | 19,57 | 41,56 | 28,10 | |
| Batch Name | CIE L Muster | CIE a Muster | CIE b Muster | CIE C Muster | CIE h Muster | CIE DE (ΔE) |
| E2 nach 6 Wäschen | 39,80 | 38,04 | 21,45 | 43,68 | 29,42 | 8,43 |

(fortgesetzt)

| Std.-Name | Std. CIE L | Std. CIE a | Std. CIE b | Std. CIE C | Std. CIE h | |
|---|---|---|---|---|---|---|
| E2 nach 10 Wäschen | 48,58 | 29,10 | 17,49 | 33,95 | 31,00 | 18,61 |
| E2 nach 12 Wäschen | 50,34 | 30,36 | 17,63 | 35,11 | 30,14 | 19,77 |
| | | | | | | |
| Std.-Name | Std. CIE L | Std. CIE a | Std. CIE b | Std. CIE C | Std. CIE h | |
| **V1** | 31,49 | 36,86 | 19,64 | 41,77 | 28,05 | |
| Batch Name | CIE L Muster | CIE a Muster | CIE b Muster | CIE C Muster | CIE h Muster | CIE DE (ΔE) |
| V1 nach 6 Wäschen | 45,17 | 35,11 | 19,96 | 40,39 | 29,62 | 13,80 |
| V1 nach 10 Wäschen | 51,62 | 28,06 | 16,88 | 32,75 | 31,03 | 22,14 |
| V1 nach 12 Wäschen | 55,57 | 23,95 | 16,41 | 29,04 | 34,42 | 27,51 |
| | | | | | | |
| Std.-Name | Std. CIE L | Std. CIE a | Std. CIE b | Std. CIE C | Std. CIE h | |
| **V2** | 34,80 | 38,05 | 21,14 | 43,53 | 29,06 | |
| Batch Name | CIE L Muster | CIE a Muster | CIE b Muster | CIE C Muster | CIE h Muster | CIE DE (ΔE) |
| V2 nach 6 Wäschen | 47,24 | 33,97 | 19,95 | 39,40 | 30,43 | 13,14 |
| V2 nach 10 Wäschen | 54,08 | 26,87 | 16,59 | 31,58 | 31,70 | 22,74 |
| V2 nach 12 Wäschen | 54,62 | 24,81 | 16,01 | 29,53 | 32,83 | 24,38 |

Versuchsreihe mit Nuance 7-65

Folgende Tönungsconditioner wurden hergestellt. E3 und E4 sind erfindungsgemäße Mittel, V3 und V4 dienen zum Vergleich. Alle Mengenangaben beziehen sich auf den Aktivsubstanzgehalt und sind in Gew.-%.

| | E3 | E4 | V3 | V4 |
|---|---|---|---|---|
| Coconut Alcohol | 4,9 | 4,9 | 4,9 | 4,9 |
| Cetearyl Alcohol | 9,7 | 9,7 | 9,7 | 9,7 |
| Propylparaben + Methylparaben | 0,6 | 0,6 | 0,6 | 0,6 |
| Cocamidopropyl Betaine | 0,7 | 0,7 | 0,7 | 0,7 |
| Ceteareth-20 | 0,9 | 0,9 | 0,9 | 0,9 |
| Polyethylengycol MG 400 (PEG-8) | 2,5 | 2,5 | 2,5 | 2,5 |
| Monoethanolamin | 0,07 | 0,07 | 0,07 | 0,07 |
| Polyquaternium-6 | 0,2 | 0,2 | 0,2 | 0,2 |
| Etidronsäure | 0,08 | 0,08 | 0,08 | 0,08 |
| Citronensäure | 0,08 | 0,08 | 0,08 | 0,08 |
| Magnesiumchlorid (wasserfrei) | 0,10* | - | - | - |
| Trimagnesiumdicitrat (wasserfrei) | - | 0,2** | - | - |

(fortgesetzt)

| | E3 | E4 | V3 | V4 |
|---|---|---|---|---|
| Natriumchlorid | 0,01 | 0,01 | 0,01 | 0,12 |
| Marulaöl | 0,2 | 0,2 | 0,2 | 0,2 |
| Keratinhydrolysat mit Mw 400-800 Da | 0,02 | 0,02 | 0,02 | 0,02 |
| D-Panthenol | 0,2 | 0,2 | 0,2 | 0,2 |
| Cocodimonium Hydroxypropyl Hydrolyzed Keratin | 0,015 | 0,015 | 0,015 | 0,015 |
| Dimethicone*** | 0,5 | 0,5 | 0,5 | 0,5 |
| Laureth-4 | 0,03 | 0,03 | 0,03 | 0,03 |
| Laureth-23 | 0,03 | 0,03 | 0,03 | 0,03 |
| Parfum | 0,7 | 0,7 | 0,7 | 0,7 |
| HC Yellow No. 2 | 0,308 | 0,308 | 0,308 | 0,308 |
| N,N-Bis(2-hydroxyethyl)-2-nitro-p-PD | 0,1995 | 0,1995 | 0,1995 | 0,1995 |
| HC Blue No. 12 | 0,399 | 0,399 | 0,399 | 0,399 |
| HC Red 3 | 0,0602 | 0,0602 | 0,0602 | 0,0602 |
| Hydroxyethyl-2-nitro-p-toluidin | 0,063 | 0,063 | 0,063 | 0,063 |
| Wasser (entmineral.) @ 100,00 Gew.-% | 77,4353 | 77,3353 | 77,5353 | 77,4253 |

* entspricht 0,026 Gew.-% $Mg^{2+}$-Ionen im Tönungsconditioner
** entspricht 0,032 Gew.-% $Mg^{2+}$-Ionen im Tönungsconditioner
*** ex Xiameter MEM-2664 Emulsion

Die farbmetrischen Daten für die Versuche mit der Nuance 7-65 sind in der folgenden Tabelle zusammengestellt:

| Std.-Name | Std. CIE L | Std. CIE a | Std. CIE b | Std. CIE C | Std. CIE h | |
|---|---|---|---|---|---|---|
| **E3** | 22,62 | 8,45 | 6,25 | 10,51 | 36,52 | |
| Batch Name | CIE L Muster | CIE a Muster | CIE b Muster | CIE C Muster | CIE h Muster | CIE DE (ΔE) |
| E3 nach 6 Wäschen | 34,32 | 11,62 | 12,21 | 16,85 | 46,42 | 13,50 |
| E3 nach 10 Wäschen | 42,76 | 11,53 | 11,87 | 16,55 | 45,81 | 21,13 |
| E3 nach 12 Wäschen | 44,23 | 11,80 | 11,38 | 16,39 | 43,97 | 22,45 |
| | | | | | | |
| Std.-Name | Std. CIE L | Std. CIE a | Std. CIE b | Std. CIE C | Std. CIE h | |
| **E4** | 23,90 | 9,79 | 9,02 | 13,31 | 42,63 | |
| Batch Name | CIE L Muster | CIE a Muster | CIE b Muster | CIE C Muster | CIE h Muster | CIE DE (ΔE) |
| E4 nach 6 Wäschen | 38,26 | 11,34 | 13,32 | 17,49 | 49,59 | 15,07 |
| E4 nach 10 Wäschen | 41,29 | 10,68 | 13,58 | 17,27 | 51,83 | 18,00 |
| E4 nach 12 Wäschen | 47,58 | 11,38 | 10,05 | 15,19 | 41,45 | 23,76 |
| | | | | | | |

(fortgesetzt)

| Std.-Name | Std. CIE L | Std. CIE a | Std. CIE b | Std. CIE C | Std. CIE h | |
|---|---|---|---|---|---|---|
| **V3** | 22,50 | 9,12 | 7,41 | 11,75 | 39,09 | |
| Batch Name | CIE L Muster | CIE a Muster | CIE b Muster | CIE C Muster | CIE h Muster | CIE DE (ΔE) |
| V3 nach 6 Wäschen | 44,35 | 11,37 | 13,21 | 17,43 | 49,28 | 22,72 |
| V3 nach 10 Wäschen | 55,20 | 11,77 | 11,39 | 16,38 | 44,08 | 33,04 |
| V3 nach 12 Wäschen | 56,38 | 11,97 | 11,97 | 16,93 | 45,02 | 34,30 |
| | | | | | | |
| Std.-Name | Std. CIE L | Std. CIE a | Std. CIE b | Std. CIE C | Std. CIE h | |
| **V4** | 23,70 | 9,38 | 7,83 | 12,22 | 39,84 | |
| Batch Name | CIE L Muster | CIE a Muster | CIE b Muster | CIE C Muster | CIE h Muster | CIE DE (ΔE) |
| V4 nach 6 Wäschen | 46,19 | 11,37 | 12,19 | 16,67 | 46,98 | 22,99 |
| V4 nach 10 Wäschen | 54,54 | 12,45 | 12,20 | 17,43 | 44,42 | 31,30 |
| V4 nach 12 Wäschen | 55,60 | 11,76 | 11,85 | 16,69 | 45,20 | 32,24 |

**Patentansprüche**

1. Kosmetisches Mittel zum gleichzeitigen semipermanenten Färben und Pflegen von Haaren, enthaltend

   (a) mindestens einen direktziehenden Farbstoff, ausgewählt aus nicht-ionischen direktziehenden Farbstoffen,
   (b) mindestens ein Alkylamidoalkylbetain in einer Gesamtmenge von 0,05 - 3 Gew.-%,
   (c) mindestens ein nichtionisches Ethoxylat-Tensid, in einer Gesamtmenge von 0,2 - 1,5 Gew.-%, bevorzugt 0,4 - 1,2 Gew.-%, besonders bevorzugt 0,6 -0,9 Gew.-%,
   (d) mindestens ein lineares 1-Alkanol mit 8 - 24 Kohlenstoffatomen in einer Gesamtmenge von 8 - 15 Gew.-%, bevorzugt 8 - 13 Gew.-% besonders bevorzugt 10 - 12 Gew.-%,
   (e) mindestens ein wasserlösliches Magnesiumsalz in einer solchen Menge, dass der Gehalt an $Mg^{2+}$-Ionen 0,005 bis 1,000 Gew.-%, bevorzugt 0,008 bis 0,500 Gew.-%, besonders bevorzugt 0,010 bis 0,200 Gew.-%, besonders bevorzugt 0,013 bis 0,100 Gew.-%, außerordentlich bevorzugt 0,018 bis 0,050 Gew.-%, beträgt,
   (f) 74 - 86 Gew.-% Wasser,
   wobei kein Alkylglycosid, kein Oxidationsfarbstoffvorprodukt und kein Oxidationsmittel enthalten sind,
   wobei der Gesamttensidgehalt 0,5 - 6 Gew.-%, bevorzugt 1 - 5 Gew.-%, besonders bevorzugt 1,5 - 3 Gew.-% beträgt,
   wobei sich alle Mengenangaben in Gew.-% auf das Gewicht des kosmetischen Mittels beziehen,
   **dadurch gekennzeichnet, dass** kein kationisches Tensid enthalten ist,
   weiterhin **dadurch gekennzeichnet, dass** das Mittel einen pH-Wert im Bereich von 2,0 bis 5,5 aufweist, gemessen bei 22°C.

2. Mittel nach Anspruch 1, **dadurch gekennzeichnet, dass** das mindestens eine Alkylamidoalkylbetain ein Alkylamidoalkylbetain der Formel (I) ist,

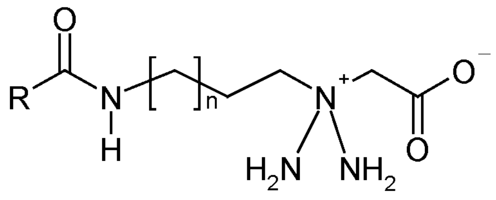

(I)

wobei

R einen linearen oder verzweigten, gesättigten oder ungesättigten Kohlenwasserstoffrest mit 7 bis 21, bevorzugt 9 bis 17 Kohlenstoffatomen, besonders bevorzugt 11 bis 13 darstellt, und
n eine ganze Zahl von 0 bis 4, insbesondere 1 bis 2 ist.

3. Mittel nach Anspruch 2, **dadurch gekennzeichnet, dass** das mindestens eine Alkylamidoalkylbetain der Formel (I) Cocamidopropylbetain ist.

4. Mittel nach einem der Ansprüche 1 bis 3, **dadurch gekennzeichnet, dass** das mindestens eine Alkylamidoalkylbetain in einer Gesamtmenge von 0,1 - 1,5 Gew.-%, bevorzugt 0,5 - 1,0 Gew.-%, enthalten ist.

5. Mittel nach einem der Ansprüche 1 bis 4, **dadurch gekennzeichnet, dass** kein anionisches Tensid enthalten ist.

6. Mittel nach einem der Ansprüche 1 bis 5, **dadurch gekennzeichnet, dass** es einen pH-Wert im Bereich von 3,5 bis 5,5, besonders bevorzugt von 4,0 bis 5,0, weiter bevorzugt von 4,1 bis 5,0 und ganz besonders bevorzugt von 4,3 bis 4,8, aufweist, jeweils gemessen bei 22°C.

7. Mittel nach einem der Ansprüche 1 bis 6, **dadurch gekennzeichnet, dass** das mindestens eine wasserlösliche Magnesiumsalz ausgewählt ist aus wasserlöslichen Magnesiumsalzen mit einem nicht-chelatisierenden Anion, bevorzugt ausgewählt aus Magnesiumchlorid, Magnesiumsulfat, Magnesiumbromid, Magnesiumbenzoat, Magnesiumformiat, Magnesiumacetat, Magnesiumpropionat und Magnesiumnitrat sowie Mischungen dieser Salze, besonders bevorzugt ausgewählt aus Magnesiumchlorid und Magnesiumsulfat sowie Mischungen hiervon; außerordentlich bevorzugt ausgewählt aus Magnesiumchlorid.

8. Mittel nach einem der Ansprüche 1 bis 7, **dadurch gekennzeichnet, dass** der mindestens eine nicht-ionische direktziehende Farbstoff ausgewählt ist aus HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, HC Blue 15, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetra-hydrochinoxalin, 2-Hydroxy-1,4-naphthochinon, N,N'-bis-(2-Hydroxyethyl)-2-nitro-p-phenylendiamin, 2-Amino-6-chloro-4-nitrophenol, 4-Ethylamino-3-nitrobenzoesäure und 2-Chlor-6-ethylamino-4-nitrophenol sowie Mischungen hiervon, bevorzugt in einer Gesamtmenge von 0,001 bis 7 Gew.-%, weiter bevorzugt von 0,01 bis 5,5 Gew.-%, weiter bevorzugt von 0,1 bis 3,4 Gew.-%, weiter bevorzugt von 0,3 bis 2 Gew.-%, jeweils bezogen auf das Gewicht des Färbemittels.

9. Mittel nach einem der Ansprüche 1 bis 8, **dadurch gekennzeichnet, dass** Natriumchlorid in einer Menge von 0 bis 0,060 Gew.-%, bevorzugt 0,005 - 0,040 Gew.-%, besonders bevorzugt 0,010 - 0,030 Gew.-%, bezogen auf das Gewicht des Färbemittels, enthalten ist.

10. Mittel nach einem der Ansprüche 1 bis 9, **dadurch gekennzeichnet, dass** Polyquaternium-6, bevorzugt in einer Menge von 0,005 bis 1 Gew.-%, besonders bevorzugt 0,01 bis 0,8 Gew.-%, stärker bevorzugt 0,05 bis 0,5 Gew.-% und insbesondere in einer Menge von 0,1 bis 0,2 Gew.-%, jeweils bezogen auf das Gewicht des Mittels, enthalten ist.

11. Mittel nach einem der Ansprüche 1 bis 10, **dadurch gekennzeichnet, dass** mindestens ein Polydimethylsiloxan mit der INCI Dimethicone enthalten ist, das außerordentlich bevorzugt in voremulgierter Form mit einer zahlenmittleren Tröpfchengröße oder Partikelgröße von 0,05 µm bis 20 µm, bevorzugt 0,5 µm bis 10 µm, enthalten ist.

12. Mittel nach einem der Ansprüche 1 bis 11, **dadurch gekennzeichnet, dass** mindestens ein pflanzliches Öl, ausgewählt aus Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannisbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl und den flüssigen Anteile des Kokosöls sowie Mischungen der vorgenannten Öle, enthalten ist, bevorzugt in einer Gesamtmenge von 0,005 bis 1 Gew.-%, besonders bevorzugt 0,01 bis 0,8 Gew.-%, stärker bevorzugt 0,05 bis 0,5 Gew.-% und insbesondere in einer Gesamtmenge von 0,1 bis 0,2 Gew.-%, jeweils bezogen auf das Gewicht des Mittels, wobei besonders bevorzugt Marulaöl enthalten ist.

13. Verfahren zur Färbung von menschlichen Haaren, bei dem ein Mittel gemäß einem der Ansprüche 1 - 12 auf die Haare appliziert und nach einer Einwirkzeit von 1 Minute bis 30 Minuten, bevorzugt 5 bis 20 Minuten bei 20 - 50 °C, bevorzugt 20 - 32 °C, ausgespült wird, optional gefolgt vom Trocknen der Haare.

## Claims

1. Cosmetic agent for the simultaneous semi-permanent coloring and conditioning of hair, containing

(a) at least one direct dye selected from nonionic direct dyes,
(b) at least one alkylamidoalkyl betaine in a total amount of 0.05-3% by weight,
(c) at least one nonionic ethoxylate surfactant in a total amount of 0.2-1.5% by weight, preferably 0.4-1.2% by weight, particularly preferably 0.6-0.9% by weight,
(d) at least one linear 1-alkanol with 8-24 carbon atoms in a total amount of 8-15 wt.%, preferably 8-13 wt.%, particularly preferably 10-12 wt.%,
(e) at least one water-soluble magnesium salt in an amount such that the content of $Mg^{2+}$ions is 0.005 to 1.000 wt.%, preferably 0.008 to 0.500 wt.%, particularly preferably 0.010 to 0.200 wt.%, particularly preferably 0.013 to 0.100 wt.%, exceptionally preferred 0.018 to 0.050 wt.%,
(f) 74-86 wt% water,
wherein no alkyl glycoside, no oxidation dye precursor, and no oxidizing agent are contained,
wherein the total surfactant content is 0.5-6 wt.%, preferably 1-5 wt.%, particularly preferably 1.5-3 wt.%,
wherein all quantities in wt% refer to the weight of the cosmetic agent,
**characterized in that** no cationic surfactant is contained,
further **characterized in that** the product has a pH value in the range of 2.0 to 5.5, measured at 22°C.

2. Agent according to claim 1, **characterized in that** the at least one alkylamidoalkyl betaine is an alkylamidoalkyl betaine of formula (I),

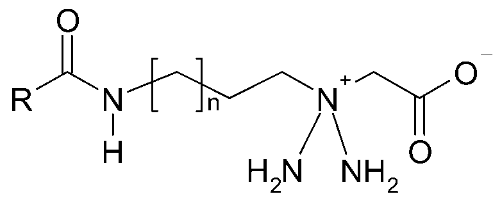

(I)

wherein

R represents a linear or branched, saturated or unsaturated hydrocarbon radical having 7 to 21, preferably 9 to 17, carbon atoms, particularly preferably 11 to 13, and
n is an integer from 0 to 4, in particular 1 to 2.

3. Agent according to claim 2, **characterized in that** the at least one alkylamidoalkyl betaine of formula (I) is cocamidopropyl betaine.

4. Agent according to one of claims 1 to 3, **characterized in that** the at least one alkylamidoalkyl betaine is contained in a total amount of 0.1-1.5 wt.%, preferably 0.5-1.0 wt.%.

5. Agent according to one of claims 1 to 4, **characterized in that** no anionic surfactant is contained.

6. Agent according to one of claims 1 to 5, **characterized in that** it has a pH value in the range from 3.5 to 5.5, particularly preferably from 4.0 to 5.0, more preferably from 4.1 to 5.0 and most preferably from 4.3 to 4.8, in each case measured at 22°C.

7. Agent according to one of claims 1 to 6, **characterized in that** the at least one water-soluble magnesium salt is selected from water-soluble magnesium salts with a non-chelating anion, preferably selected from magnesium chloride, magnesium sulfate, magnesium bromide, magnesium benzoate, magnesium formate, magnesium acetate, magnesium propionate, and magnesium nitrate, as well as mixtures of these salts, particularly preferably selected from magnesium chloride and magnesium sulfate, as well as mixtures thereof; exceptionally preferably selected from magnesium chloride.

8. Agent according to any one of claims 1 to 7, **characterized in that** the at least one nonionic direct dye is selected from HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, HC Blue 15, Disperse Blue 3, HC Violet 1, Disperse Violet 1, Disperse Violet 4, Disperse Black 9, 1,4-Diamino-2-nitrobenzene, 2-Amino-4-nitrophenol, 1,4-Bis-(2-hydroxyethyl)-amino-2-nitrobenzene, 3-Nitro-4-(2-hydroxyethyl)aminophenol, 2-(2-Hydroxyethyl)amino-4,6-dinitrophenol, 4-[(2-hydroxyethyl)amino]-3-nitro-1-methylbenzene, 1-amino-4-(2-hydroxyethyl)amino-5-chloro-2-nitrobenzene, 4-amino-3-nitrophenol, 1-(2'-ureidoethyl)amino-4-nitrobenzene, 2-[(4-amino-2-nitrophenyl)amino]benzoic acid, 4-[(3-hydroxypropyl)amino]-3-nitrophenol, 4-nitro-o-phenylenediamine, 6-nitro-1,2,3,4-tetrahydroquinoxaline, 2-hydroxy-1,4-naphthoquinone, picramic acid and its salts, N,N'-bis-(2-hydroxyethyl)-2-nitro-p-phenylenediamine, 2-amino-6-chloro-4-nitrophenol, 4-ethylamino-3-nitrobenzoic acid and 2-chloro-6-ethylamino-4-nitrophenol, as well as mixtures thereof, preferably in a total amount of 0.001 to 7 wt.%, more preferably from 0.01 to 5.5 wt.%, more preferably from 0.1 to 3.4 wt.%, more preferably from 0.3 to 2 wt.%, in each case based on the weight of the dye.

9. Agent according to one of claims 1 to 8, **characterized in that** sodium chloride is contained in an amount of 0 to 0.060 wt.%, preferably 0.005 to 0.040 wt.%, particularly preferably 0.010 to 0.030 wt.%, based on the weight of the dye.

10. Agent according to one of claims 1 to 9, **characterized in that** polyquaternium-6 is contained, preferably in an amount of 0.005 to 1 wt.%, more preferably 0.01 to 0.8 wt.%, more strongly preferred 0.05 to 0.5 wt.% and in particular in an amount of 0.1 to 0.2 wt.%, in each case based on the weight of the agent.

11. Agent according to one of claims 1 to 10, **characterized in that** at least one polydimethylsiloxane with the INCI dimethicone is contained, which is extremely preferably contained in pre-emulsified form with a number-average droplet size or particle size of 0.05 $\mu$m to 20 $\mu$m, preferably 0.5 $\mu$m to 10 $\mu$m.

12. Composition according to any of claims 1 to 11, **characterized in that** at least one vegetable oil selected from amaranth seed oil, apricot kernel oil, argan oil, avocado oil, babassu oil, cottonseed oil, borage seed oil, camelina oil, safflower oil, peanut oil, pomegranate seed oil, grapefruit seed oil, hemp oil, hazelnut oil, elderberry seed oil, currant seed oil, jojoba oil, linseed oil, macadamia nut oil, corn oil, almond oil, marula oil, evening primrose oil, olive oil, palm oil, palm kernel oil, Brazil nut oil, pecan nut oil, peach kernel oil rapeseed oil, castor oil, sea buckthorn pulp oil, sea buckthorn kernel oil, sesame oil, soybean oil, sunflower oil, grape seed oil, walnut oil, wild rose oil, wheat germ oil and the liquid components of coconut oil, as well as mixtures of the aforementioned oils, preferably in a total amount of 0.005 to 1% by weight, particularly preferably 0.01 to 0.8% by weight, more preferably 0.05 to 0.5% by weight and especially in a total amount of 0.1 to 0.2% by weight, in each case based on the weight of the agent, wherein marula oil is particularly preferred.

13. Method for coloring human hair, in which an agent according to one of claims 1-12 is applied to the hair and, after an exposure time of 1 minute to 30 minutes, preferably 5 to 20 minutes at 20-50°C, preferably 20-32°C, is rinsed out, optionally followed by drying the hair.

**Revendications**

1. Produit cosmétique pour la coloration semi-permanente et le soin des cheveux, contenant

(a) au moins un colorant direct choisi parmi les colorants directs non ioniques,
(b) au moins une alkylamidoalkylbétaïne en une quantité totale de 0,05 à 3 % en poids,
(c) au moins un tensioactif éthoxylé non ionique, en une quantité totale de 0,2 à 1,5 % en poids, de préférence de 0,4 à 1,2 % en poids, de manière particulièrement préférée de 0,6 à 0,9 % en poids,
(d) au moins un 1-alcanol linéaire comportant 8 à 24 atomes de carbone en une quantité totale comprise entre 8 et 15 % en poids, de préférence entre 8 et 13 % en poids, de manière particulièrement préférée entre 10 et 12 % en poids,
(e) au moins un sel de magnésium soluble dans l'eau en une quantité telle que la teneur en ions $Mg^{2+}$ soit comprise entre 0,005 et 1,000 % en poids, de préférence entre 0,008 et 0,500 % en poids, de manière particulièrement préférée entre 0,010 et 0,200 % en poids, de manière particulièrement préférée entre 0,013 et 0,100 % en poids, très préférentiellement de 0,018 à 0,050 % en poids,
(f) 74 à 86 % en poids d'eau,
sans contenir d'alkylglycoside, de précurseur de colorant d'oxydation ni d'agent oxydant,
la teneur totale en tensioactifs étant comprise entre 0,5 et 6 % en poids, de préférence entre 1 et 5 % en poids, de manière particulièrement préférée entre 1,5 et 3 % en poids,
toutes les quantités exprimées en % en poids se rapportant au poids du produit cosmétique, **caractérisé en ce qu'**il ne contient pas de tensioactif cationique,
en outre **caractérisé en ce que** le produit présente un pH compris entre 2,0 et 5,5, mesuré à 22 °C.

**2.** Produit selon la revendication 1, **caractérisé en ce que** la au moins une alkylamidoalkylbétaïne est une alkylamidoalkylbétaïne de formule (I)

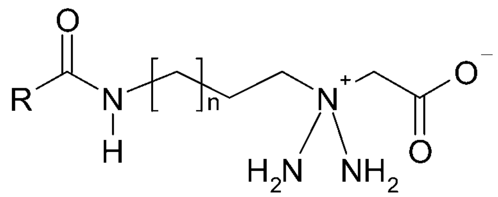

(I)

dans laquelle

R représente un radical hydrocarboné linéaire ou ramifié, saturé ou insaturé, comportant 7 à 21, de préférence 9 à 17 atomes de carbone, de manière particulièrement préférée 11 à 13, et
n est un nombre entier de 0 à 4, en particulier de 1 à 2.

**3.** Produit selon la revendication 2, **caractérisé en ce que** la au moins une alkylamidoalkylbétaïne de formule (I) est la cocamidopropylbétaïne.

**4.** Produit selon l'une des revendications 1 à 3, **caractérisé en ce que** l'au moins une alkylamidoalkylbétaïne est contenue en une quantité totale de 0,1 à 1,5 % en poids, de préférence de 0,5 à 1,0 % en poids.

**5.** Produit selon l'une des revendications 1 à 4, **caractérisé en ce qu'**il ne contient pas de tensioactif anionique.

**6.** Produit selon l'une des revendications 1 à 5, **caractérisé en ce qu'**il présente un pH compris entre 3,5 et 5,5, de préférence entre 4,0 et 5,0, de préférence encore entre 4,1 et 5,0 et de préférence tout particulièrement entre 4,3 et 4,8, mesuré à chaque fois à 22 °C.

**7.** Produit selon l'une des revendications 1 à 6, **caractérisé en ce que** le au moins un sel de magnésium hydrosoluble est choisi parmi les sels de magnésium hydrosolubles ayant un anion non chélatant, de préférence choisi parmi le chlorure de magnésium, le sulfate de magnésium, le bromure de magnésium, le benzoate de magnésium, le formiate de magnésium, l'acétate de magnésium, le propionate de magnésium et le nitrate de magnésium, ainsi que des mélanges de ces sels, de préférence choisis parmi le chlorure de magnésium et le sulfate de magnésium, ainsi que des mélanges de ceux-ci ; de manière particulièrement préférée, choisi parmi le chlorure de magnésium.

**8.** Produit selon l'une des revendications 1 à 7, **caractérisé en ce que** le au moins un colorant non ionique à fixation directe est choisi parmi HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC

Blue 12, HC Blue 15, Disperse Blue 3, HC Violet 1, Disperse Violet 1, Disperse Violet 4, Disperse Black 9, 1,4-Diamino-2-nitrobenzène, 2-Amino-4-nitrophénol, 1,4-Bis-(2-hydroxyéthyl)-amino-2-nitrobenzène, 3-nitro-4-(2-hydroxyéthyl)aminophénol, 2-(2-hydroxyéthyl)amino-4,6-dinitrophénol, 4-[(2-hydroxyéthyl)amino]-3-nitro-1-méthylbenzène, 1-amino-4-(2-hydroxyéthyl)amino-5-chloro-2-nitrobenzène, 4-amino-3-nitrophénol, 1-(2'-uréidoéthyl)amino-4-nitrobenzène, acide 2-[(4-amino-2-nitrophényl)amino]benzoïque, 4-[(3-hydroxypropyl)amino]-3-nitrophénol, 4-nitro-o-phénylènediamine, 6-nitro-1,2,3,4-tétrahydroquinoxaline, 2-hydroxy-1,4-naphtoquinone, acide picramique et ses sels, N,N'-bis-(2-hydroxyéthyl)-2-nitro-p-phénylènediamine, 2-amino-6-chloro-4-nitrophénol, acide 4-éthylamino-3-nitrobenzoïque et 2-chloro-6-éthylamino-4-nitrophénol ainsi que leurs mélanges, de préférence en une quantité totale de 0,001 à 7 % en poids, de préférence encore de 0,01 à 5,5 % en poids, de préférence encore de 0,1 à 3,4 % en poids, de préférence encore de 0,3 à 2 % en poids, dans chaque cas par rapport au poids du colorant.

**9.** Produit selon l'une des revendications 1 à 8, **caractérisé en ce qu'**il contient du chlorure de sodium en une quantité de 0 à 0,060 % en poids, de préférence de 0,005 à 0,040 % en poids, de manière particulièrement préférée de 0,010 à 0,030 % en poids, par rapport au poids du colorant.

**10.** Produit selon l'une des revendications 1 à 9, **caractérisé en ce qu'**il contient du polyquaternium-6, de préférence en une quantité de 0,005 à 1 % en poids, de préférence encore de 0,01 à 0,8 % en poids, de préférence encore plus fortement de 0,05 à 0,5 % en poids et en particulier en une quantité de 0,1 à 0,2 % en poids, dans chaque cas par rapport au poids du produit.

**11.** Produit selon l'une des revendications 1 à 10, **caractérisé en ce qu'**il contient au moins un polydiméthylsiloxane avec l'INCI Dimethicone, qui est contenu de manière extrêmement préférée sous forme préémulsionnée avec une taille moyenne en nombre de gouttelettes ou de particules de 0,05 $\mu$m à 20 $\mu$m, de préférence de 0,5 $\mu$m à 10 $\mu$m.

**12.** Produit selon l'une des revendications 1 à 11, **caractérisé en ce qu'**il contient au moins une huile végétale choisie parmi l'huile de graines d'amarante, l'huile de noyau d'abricot, l'huile d'argan, l'huile d'avocat, l'huile de babassu, l'huile de coton, l'huile de graines de bourrache, huile de caméline, huile de carthame, huile d'arachide, huile de pépins de grenade, huile de pépins de pamplemousse, huile de chanvre, huile de noisette, huile de pépins de sureau, huile de pépins de cassis, huile de jojoba, huile de lin, huile de noix de macadamia, huile de germes de maïs, huile d'amande, huile de marula, huile d'onagre, huile d'olive, huile de palme, huile de palmiste, huile de noix du Brésil, huile de noix de pécan, huile de noyau de pêche, huile de colza, huile de ricin, huile de pulpe d'argousier, huile de pépins d'argousier, huile de sésame, huile de soja, huile de tournesol, huile de pépins de raisin, huile de noix, huile de rose sauvage, huile de germe de blé et les parties liquides de l'huile de coco, ainsi que des mélanges des huiles susmentionnées, de préférence dans une quantité totale de 0,005 à 1 % en poids, de préférence de 0,01 à 0,8 % en poids, de préférence encore de 0,05 à 0,5 % en poids et en particulier dans une quantité totale de 0,1 à 0,2 % en poids, par rapport au poids du produit, l'huile de marula étant particulièrement préférée.

**13.** Procédé de coloration des cheveux humains, dans lequel un produit selon l'une des revendications 1 à 12 est appliqué sur les cheveux et, après un temps de pose de 1 à 30 minutes, de préférence de 5 à 20 minutes à une température de 20 à 50 °C, de préférence de 20 à 32 °C, est rincé, éventuellement suivi d'un séchage des cheveux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 200202062 A1
- DE 102011089223 A1
- DE 102017223420 A1
- WO 2002100363 A2